(19) Europäisches Patentamt
European Patent Office
Office européen des brevets

(11) Publication number: 0 198 865
B1

(12) **EUROPEAN PATENT SPECIFICATION**

(45) Date of publication of patent specification: **23.05.90**

(21) Application number: **85905074.2**

(22) Date of filing: **11.10.85**

(86) International application number:
**PCT/GB85/00459**

(87) International publication number:
**WO 86/02348 24.04.86 Gazette 86/09**

(51) Int. Cl.$^5$: **C 07 C 67/14,** C 07 C 69/78, C 07 C 69/83, C 07 C 69/92, C 07 C 69/96, C 07 C 327/20, C 07 F 7/22

# (54) PREPARATION OF MONOMERS.

(30) Priority: **11.10.84 US 659599**
**27.08.85 GB 8521324**

(43) Date of publication of application:
**29.10.86 Bulletin 86/44**

(45) Publication of the grant of the patent:
**23.05.90 Bulletin 90/21**

(84) Designated Contracting States:
**AT BE CH DE FR GB IT LI NL SE**

(56) References cited:
**EP-A-0 029 362**
**EP-A-0 056 501**
**DE-A-2 939 782**
**FR-A-1 058 341**
**US-A-4 129 594**

**Chemical Abstracts, vol. 77, no. 21, 20 November 1972 (Columbus, Ohio, US) see page 407, column 2, abstract no. 139625r**

(73) Proprietor: **RAYCHEM LIMITED**
**Rolls House 7, Rolls Buildings Fetter Lane**
**London, EC4 1NL (GB)**

(72) Inventor: **TOWLE, Ian, David, Henderson**
**2 Barn Way Stratton, Cirencester**
**Gloucester GL7 2NA (GB)**

(74) Representative: **Jay, Anthony William et al**
**Raychem Limited Intellectual Property Law**
**Department Faraday Road**
**Dorcan Swindon Wiltshire (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 198 865 B1

**Description**

This invention relates to the preparation of organic compounds and especially to the preparation of monomers for subsequent use in polymerisation reactions.

Known industrial methods for producing monomers, especially aromatic monomers wherein an aromatic group is bonded directly to a functional group such as an ester, thioester or carbonate, often have a number of disadvantages. For example high temperatures are usually required and/or unpleasant solvents such as pyridine are necessary. Aromatic ester monomers, for example can be prepared using a phase-transfer catalysis method, but this method has the disadvantage that two or more recrystallisations of the product are usually necessary in order to obtain a satisfactorily pure monomer. Preparation of aromatic esters by condensation of aryl chlorides with phenols as described in DE—A—2939789 and US—A—4129594.

In a paper by J Valade and M Pereyre entitled 'Étude de la scission de la liaison Sn—O—C dans les monoalcoxytrialcoyétains', Report de l'Academic des Sciences (FR), Chimie Organique, Seance du, 21 Mai 1962, pp. 3693 there is described a method for making simple esters by reacting methoxytributyltin with acetyl chloride or benzoyl chloride, the reaction producing methyl acetate or methyl benzoate. The use of tin in intermediates for making polymers is described in European Patent Publication No. 0,154,506.

In a first aspect the present invention provides a method for the preparation of an organic compound comprising reacting a first compound of the formula:

$$(R)_r—M—Y—A^1—Y—M—(R)_r$$

where

each R is independently a substituted or unsubstituted alkyl or aryl group;

each r is independently an integer from 1 to 4 inclusive depending upon the element M used;

each M is independently an element selected from Group IIIB, IVB or VB of the Periodic Table (IUPAC 1965 revision) or a transition metal, excluding carbon, silicon, nitrogen, phosphorus, boron, aluminium and titanium;

each Y is independently an oxygen atom, a sulphur atom, a substituted nitrogen atom other than

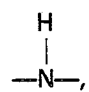,

or a substituted phoshorous atom other than

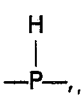, 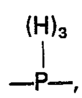, or 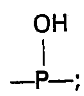;

and $A^1$ is at least partly aromatic with each atom Y bonded to an aromatic group of $A^1$, with a second compound of the formula:

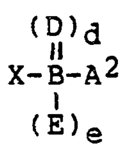

where

X is a halogen atom or a group capable of reacting with the first compound to eliminate a compound containing M and X;

B is an atom selected from carbon, phosphorus, sulphur or silicon;

D is an oxygen or sulphur atom or an amine group;

d is 1 if B is carbon, zero or 1 if B is phosphorus, zero, 1 or 2 if B is sulphur or zero if B is silicon;

E is selected from an aromatic group, aliphatic group, OR' or $NR'_2$ if B is phosphorus, or from an aromatic group, aliphatic group or —OR' if B is silicon, where R' is a substituted or unsubstituted alkyl or aryl group;

e is zero if B is carbon or sulphur, 1 if B is phosphorus or 2 if B is silicon; and

$A^2$ is an aromatic, aliphatic, aromatic/aliphatic, heterocyclic, alicyclic, siloxyl or silane group, to eliminate the compound $(R)_rMX$ and produce a compound of the formula:

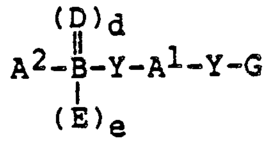

where G is either the group —M—$(R)_r$, or the group

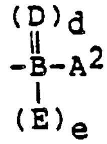

Preferably the stoichiometric ratio of the first compound to the second compound is 1:2 to produce a compound, usually a monomer, of the formula:

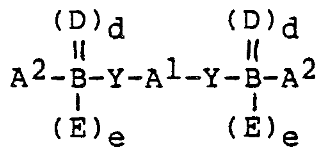

An example of this reaction is as follows:

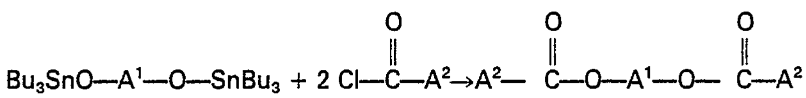

In a second aspect of the present invention provides a method for the preparation of an organic compound comprising reacting a first compound of the formula:

$$(R)_r—M—Y—A^1—Y—M—(R)_r$$

where

each R is independently a substituted or unsubstituted alkyl or aryl group;

each r is independently an integer from 1 to 4 inclusive depending upon the element M used;

each M is independently an element selected from Group IIIB, IVB or VB of the Periodic Table (IUPAC 1965 revision) or a transition metal, excluding carbon, silicon, nitrogen, phosphorus, boron, aluminium and titanium;

each Y is independently an oxygen atom, a sulphur atom, a substituted nitrogen atom other than

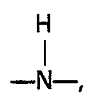

or a substituted phoshorous atom other than

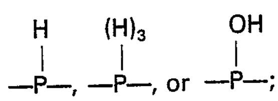

and $A^1$ is at least partly aromatic with each atom Y bonded to an aromatic group of $A^1$, with a second compound of the formula:

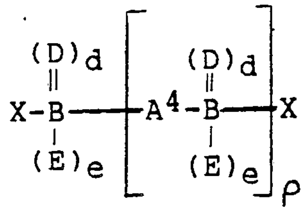

where

X is a halogen atom or a group capable of reacting with the first compound to eliminate a compound

containing M and X;

B is an atom selected from carbon, phosphorus, sulphur or silicon;

D is an oxygen or sulphur atom or an amine group;

d is 1 if B is carbon, zero or 1 if B is phosphorus, zero, 1 or 2 if B is sulphur or zero if B is silicon;

E is selected from an aromatic group, aliphatic group, or OR' or $NR'_2$ if B is phosphorus, or from an aromatic group, aliphatic group or —OR' if B is silicon, where R' is a substituted or unsubstituted alkyl or aryl group;

e is zero if B is carbon or sulphur, 1 if B is phosphorus or 2 if B is silicon; and

p is zero or 1,

to eliminate thecompound $(R)_rMX$ and produce a compound of the formula:

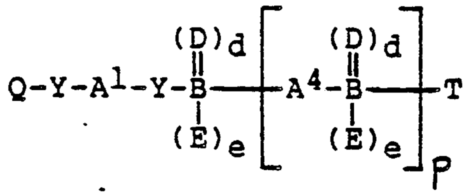

where Q is the either the group $(R)_rM$—, or the group

group 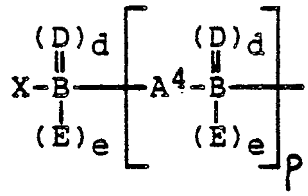 ;and

T is either the atom or group X, or the group —Y—$A^1$—Y—M—$(R)_r$.

The method according to this second aspect is especially useful when it is desired to produce a monomer having reactive end groups of the type $(R)_rM$— or

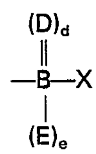

which can then be used directly in polymerisation reactions. An especially preferred monomer is one having acid chloride,

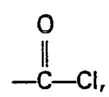

end groups.

When carrying out the method according to the second aspect of the invention, the reaction conditions should be selected so as to prevent a polymerisation reaction between the first and second compounds. Therefore it is preferred to add a solution of the first compound gradually, for example dropwise, to a solution of the second compound. Also it is preferred that the reaction is carried out in a solvent in which the product is substantially insoluble so that the product precipitates out of solution once it is formed.

When p is zero, the second compound being phosgene for example, the reaction produces a compound wherein the group

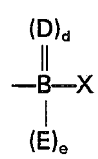

is bonded at both sides to a Y atom, such as in a carbonate. However, it is generally preferred that p is 1 and examples of reactions according to the second aspect of the invention are as follows:

(a) 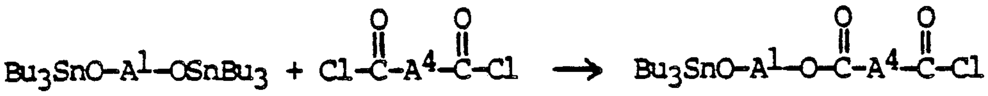

(b) 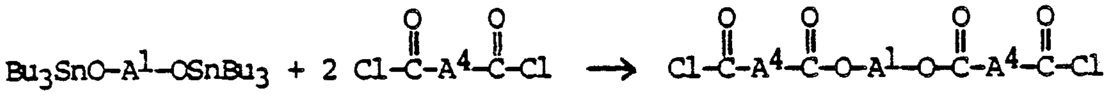

(c) 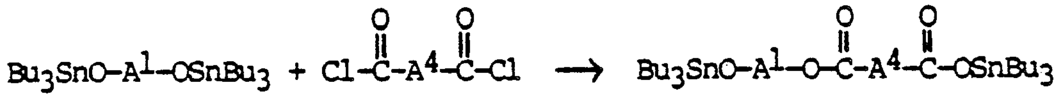

The first compound may also be 'polymeric' and thus a third aspect of the present invention provides a method for the preparation of an organic compound comprising reacting a first compound of the formula:

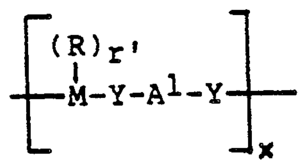

where

R is independently a substituted or unsubstituted alkyl or aryl group;

r' is independently zero or an integer from 1 to 3 depending upon the element M used;

each Y is independently an oxygen atom, a sulphur atom, a substituted nitrogen atom other than

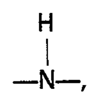

or a substituted phoshorous atom other than

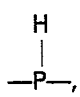 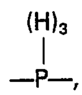 or 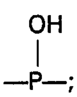;

$A^1$ is at least partly aromatic with each atom Y bonded to an aromatic group of $A^1$,

x is an integer greater than 1,

with a second compound of the formula:

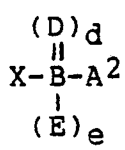

where

X is a halogen atom or a group capable of reacting with the first compound to eliminate a compound containing M and X;

B is an atom selected from carbon, phosphorus, sulphur or silicon;

D is an oxygen or sulphur atom or an amine group;

d is 1 if B is carbon, zero or 1 if B is phosphorus, zero, 1 or 2 if B is sulphur or zero if B is silicon;

E is selected from an aromatic group, aliphatic group, or OR' or $NR'_2$ if B is phosphorus, or from an aromatic group, aliphatic group or —OR' if B is silicon, where R' is a substituted or unsubstituted alkyl or aryl group;

e is zero if B is carbon or sulphur, 1 if B is phosphorus or 2 if B is silicon; and

$A^2$ is an aromatic, aliphatic, aromatic/aliphatic, heterocyclic, alicyclic, siloxyl or silane group,

to eliminate the compound

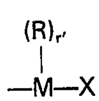

and produce a compound of the formula:

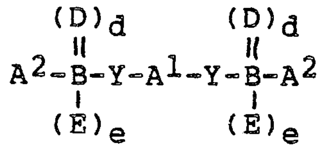

Many different types of compounds can be prepared by the method according to any of the above aspects of the invention. These include, for example, esters, thioesters, amides, thioamides, imides, thioimides, carbonates, thiocarbonates and urethanes, but preferably esters, thioesters, carbonates or thiocarbonates are prepared. Therefore it is preferred that the group

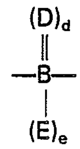

is a carbonyl

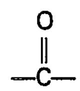 group

or a thiocarbonyl

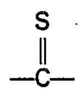 group,

more preferably a carbonyl group, and that the atom Y is an oxygen or sulphur atom, more preferably an oxygen atom. The methods of the invention are especially advantageous for the preparation of aromatic esters, aromatic thioesters, aromatic carbonates or aromatic thiocarbonates, these being difficult to produce by known commercial methods. By 'aromatic ester', 'aromatic thioester', etc., is meant a compound containing an ester group, thioester group, carbonate group or thiocarbonate group bonded to an aromatic group, for example

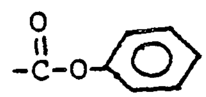.

It is especially preferred that the compound produced by the reaction contains an ester, thioester, carbonate or thiocarbonate group that is bonded on both sides to an aromatic group; that is to say the resulting compound contains, for example, an aromatic ester group

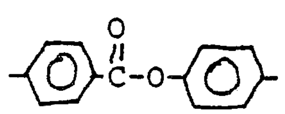.

Aromatic esters, thioesters, carbonates or thiocarbonates may be prepared by reacting a difunctional compound containing two $(R)_rM$— groups or a polymeric compound containing the repeat unit

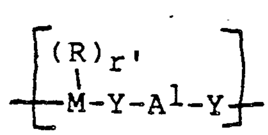

as defined in the above aspects of the invention, or they may be prepared by reacting a monofunctional compound containing only one $(R)_rM$— group with a second compound which may be either mono- or difunctional.

Accordingly, in a fourth aspect the present invention provides a method for the preparation of an aromatic ester, aromatic thioester, aromatic carbonate or aromatic thiocarbonate compound comprising reacting a first compound of the formula:

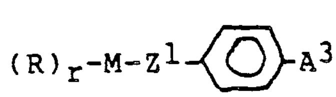

where

each R is independently a substituted or unsubstituted alkyl or aryl group;

r is an integer from 1 to 4 inclusive depending upon the element M used;

M is an element selected from Group IIIB, IVB or VB of the Periodic Table or a transition metal, excluding carbon, silicon, nitrogen, phosphorus, boron, aluminium and titanium;

$Z^1$ is an oxygen or a sulfur atom, and;

$A^3$ is an aromatic, aliphatic, aromatic/aliphatic, heterocyclic, alicyclic, siloxyl or silane group, or a hydrogen atom

with a second compound of the formula:

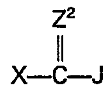

where

X is a halogen atom or a group capable of reacting with the first compound to eliminate a compound containing M and X;

$Z^2$ is either an oxygen or a sulphur atom; and

J is eitehr the atom or group X or the group $A^2$ where $A^2$ is an aromatic, aromatic/aliphatic, heterocyclic, alicyclic, siloxyl or silane group, or the group

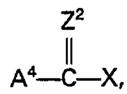

where $A^4$ is an aromatic, aliphatic, aromatic/aliphatic, heterocyclic, alicyclic, siloxyl or silane group, to eliminate the compound $(R)_rMX$ and produce an aromatic ester, aromatic thioester, aromatic carbonate or aromatic thiocarbonate compound of the formula:

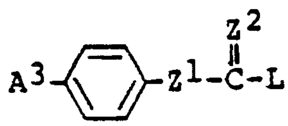

where L is either the atom or group X, the group $A^2$, the group

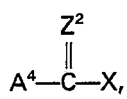

or the group

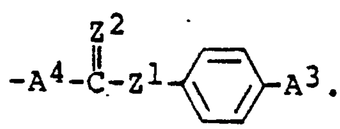

The preferred stoichiometric ratio of the first compound to the second compound according to the fourth aspect of the invention is either 1:1 or 2:1. If a stoichiometric ratio of 1:1 is chosen the second compound may be of the formula:

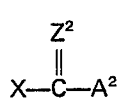

and the reaction of the two compounds produces a compound of the formula:

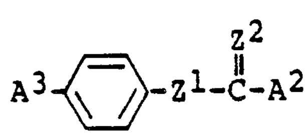

An example of this reaction is:

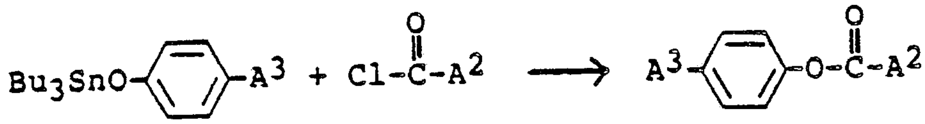

Alternatively the second compound may be of the formula:

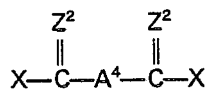

to produce, again using a stoichiometric ratio of first to second compounds of 1:1, a compound of the formula:

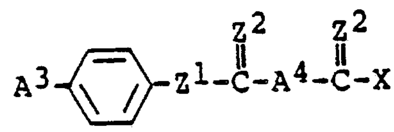

An example of this reaction is:

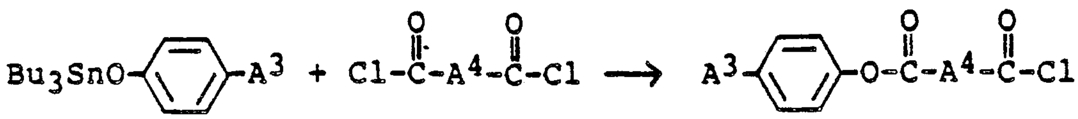

To prevent the product from reacting with a further molecule of the first compound it is preferred that the first and second compounds are reacted together by gradually adding, for example in drops, a solution of the first compound to a solution of the second compound, and also that the reaction is carried out in a solvent in which the compound produced by the reaction is substantially insoluble so that it precipitates out of solution once it is formed.

In another alternative the second compound may be of the formula

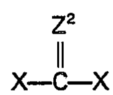

to produce a monomer of the formula:

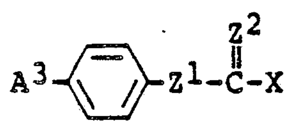

An example of this reaction is:

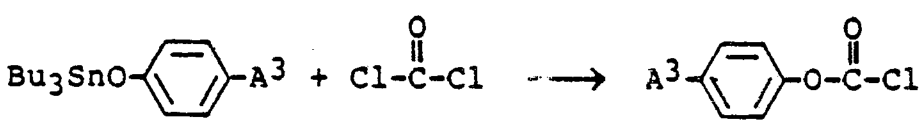

If a stoichiometric ratio of 2:1 is used in the method according to the fourth aspect of the invention, the second compound is preferably of the formula:

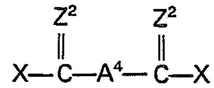

the reaction of first and second compounds then producing a monomer of the formula:

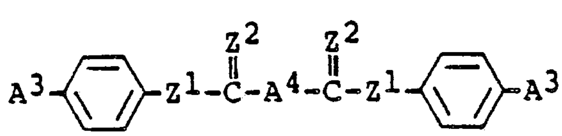

An example of this reaction is:

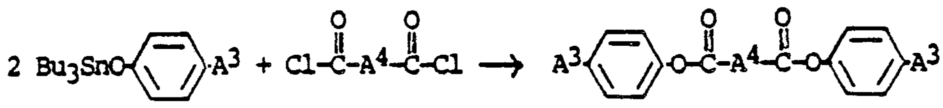

Preferably in the resulting compound the

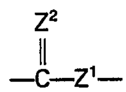

group is bonded on both sides to an aromatic group. Therefore it is preferred that the $A^2$ and $A^4$ groups in the second compounds are each at least partly aromatic, and the

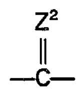

group is bonded to an aromatic group of the $A^2$ and $A^4$ group. Thus the compound produced by the reaction preferably contains a group of the formula

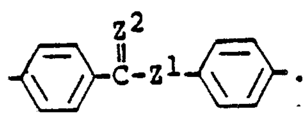

Preferably both $Z^1$ and $Z^2$ are oxygen atoms, the reaction between the first and second compounds therefore producing an aromatic ester.

Hereinafter the description of the invention relates to all aspects of the invention unless otherwise specified.

The method according to the invention is especially advantageous for the preparation of compounds wherein at least one end group, and preferably both end groups, are aromatic, for example a phenyl group. Such compounds are useful as monomers which can be polymerised with one or more other types of monomers, or with themselves, to form, for example, polyesters or polyetheresters. However, the method according to the invention may also be used for the preparation of compounds which are not for subsequent use in polymerisation reactions.

One advantage of the invention is that compounds can often be prepared using relatively low reaction temperatures, usually between −30 degrees and 150 degrees C (although it is to be understood that higher and lower temperatures can also be used). The use of low temperatures enables thermally sensitive groups to be included in the compound, for example carbon-carbon double and triple bonds.

Another advantage is that the reagents and solvent sused in the reaction can be chosen to be relatively non-hazardous. Also the eliminated compounds or by-product, $(R)_rMX$, of the reaction is generally soluble in many solvents, making purification of the resulting compound an unusually easy task. Furthermore this by-product may form a starting reagent for the preparation of the first compound.

Preferably the element M in the first compound is selected from Group IVB of the Periodic Table and is in oxidation state (+4). More preferably M is either tin or germanium, and tin (+4) is especially preferred.

Suitable groups for R include unsubstituted alkyl groups such as methyl, ethyl, propyl, butyl, pentyl, octyl, etc., substituted alkyl groups such as benzyl or phenylethyl, or a substituted or unsubstituted aryl group such as phenyl, naphthyl or biphenyl. Preferably, however, R is an alkyl group containing 3 or 4 carbon atoms, an n-butyl group being especially preferred. The number, r, of R groups attached to the element M depends upon the valency of the element M; for example when M is tin (+4) then r is 3. Thus in the first and second aspects of the invention the first compound preferably has the formula $Bu_3Sn—Y—A^1—SnBu_3$, and in the third aspect the first compound preferably has the formula

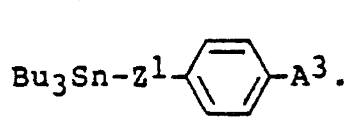

There are many groups suitable for use as the $A^1$, $A^2$, $A^3$ and $A^4$ groups in the first and second compounds. Subject to $A^1$ being at least partly aromatic as stated above these are selected from an aromatic, aliphatic, aromatic/aliphatic, heterocyclic, alicyclic, siloxyl or silane group, or for $A^3$ a hydrogen atom. This includes substituted or unsubstituted groups, heteroaromatic, heteroaliphatic and multiple

aromatic groups which may be joined by an oxygen or sulphur atom or sulphone, imide or ketone group for example. The $A^1$ and $A^4$ groups are difunctional, the $A^1$ group being bonded at each end to a Y atom or group, and the $A^4$ group being bonded at each end to a

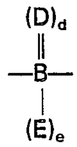 group or 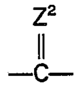 group.

The $A^2$ and $A^3$ groups are monofunctional and, as mentioned above, it is preferred that the end of the $A^2$ or $A^3$ group remote from the B or $Z^1$ atom respectively is aromatic. The following list gives examples of suitable groups for the monofunctional $A^2$ and $A^3$ groups. Similar groups are also suitable for $A^1$ and $A^4$ by removal of a hydrogen atom, thus providing a difunctional group.

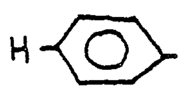 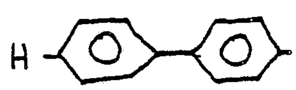 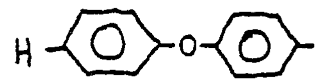

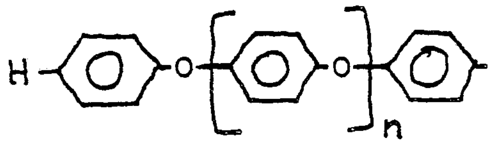 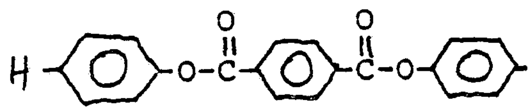

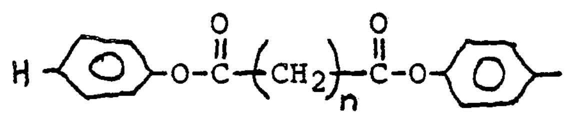

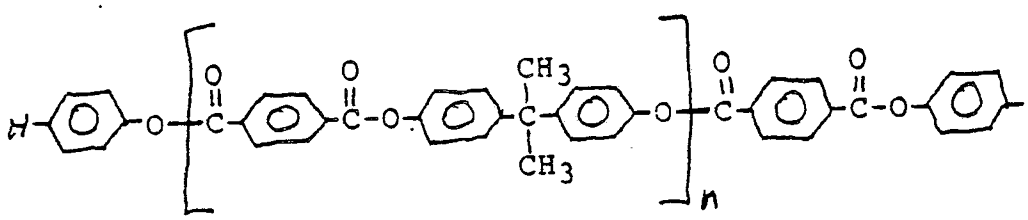

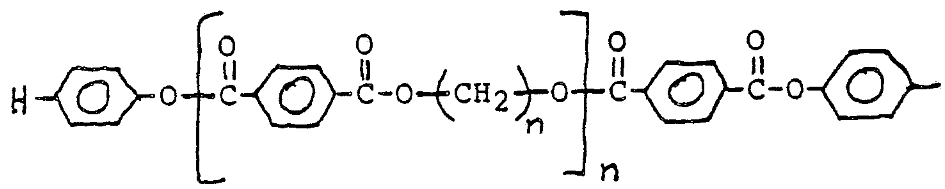

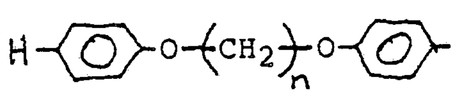 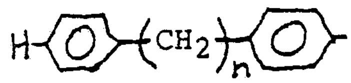 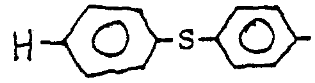

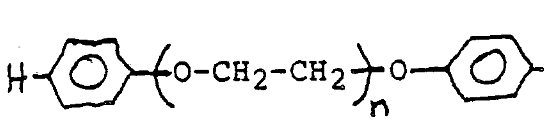 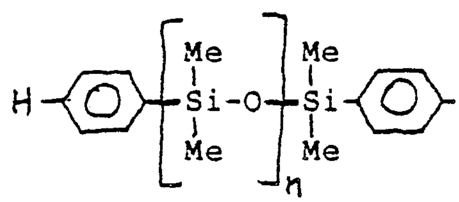

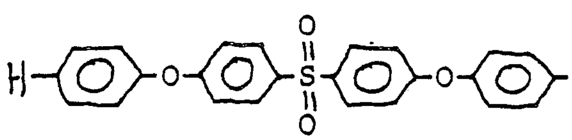 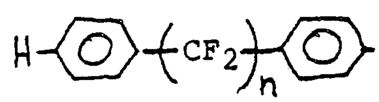

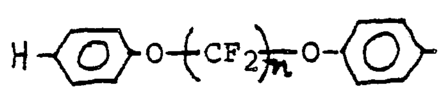

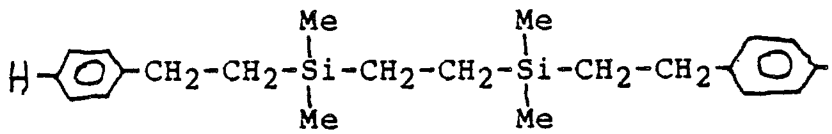

These examples listed for the A groups are bonded to the B or Y atom with an aromatic carbon atom. However groups may also be chosen for any one of the A groups except $A^1$ wherein an aliphatic carbon atom or a silicon atom is bonded to the Y atom of the first compound or the B or C atom of the second compound as appropriate. Also the $A^2$ and $A^4$ groups may contain an oxygen, nitrogen, or sulphur for example which bonds to the B or C atom respectively in the second compound in the first, second or third aspects of the invention.

Typical solvents used in the process of the present invention include, for example, chloroform, xylene, toluene, tetrahydrofuran, chlorobenzene, 1,2-dichloroethane, benzophenone, diphenylsulphone, or mixtures thereof, although it should be remembered that when the compound produced has end groups of the formula $(R)_rM$— or —X then the solvent chosen should be one in which the compound produced is substantially insoluble.

Once the compound has been isolated it is possible for the by-product to be easily removed, for example by extraction with acetone, hexane, methanol or other simple solvents which do not affect the resulting compound adversely. The by-product can then be recovered by distillation or crystallization for example.

There are a number of possible routes for preparing the first compound for the method according to the invention. In the first aspect of the invention the first compound, for example $Bu_3SnO—A^1—OSnBu_3$, may be prepared by reaction of $Bu_3SnOMe$ with $HO—A^1—OH$. Similarly in the second aspect of the invention the first compound, for example

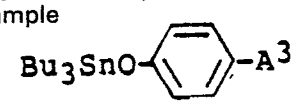

may be prepared by reaction of $Bu_3SnOMe$ with

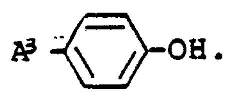

The invention will now be illustrated by the following examples.

Example 1

To 400 mls of xylene was added 50 g of thiophenol and 135.3 g of bis(tributyltin)oxide. The reaction mixture was heated to boiling and water of reaction removed using a Dean-Stark head. After all the water had been removed heating was discontinued and when refluxing had subsided 46.06 g of isophthaloyl dichloride added. This produced a slight yellowing of the solution. The mixture was then heated to reflux for 3 hours and then allowed to cool. The colourless crystalline product was collected by filtration, washed with cold toluene and dried. A further crop of crystals was obtained by concentration the mother liquors.

Yield was 76.5 g, 96%.

The product was identified as

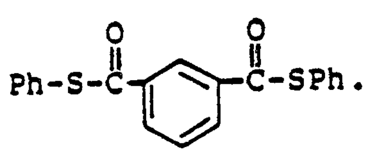

The analogous terephthaloyl product was prepared by repeating the above reaction, replacing the isophthaloyl dichloride with terephthaloyl dichloride.

Example 2

To 225 mls of xylene was added 5.50 g of hydroquinone and 29.81 g of bis(tributyltin)oxide. The reaction mixture was heated to boiling and water of reaction removed using a Dean-Stark head. After all the water has been removed heating was discontinued and when refluxing has subsided 24.43 g of 4-phenoxybenzoylchloride added. The mixture was then heated to reflux for 3 hours and then allowed to cool. The colourless crystalline product was collected by filtration, washed with cold toluene and dried. A further crop of crystals was obtained by concentrating the mother liquors.

Yield was 24.0 g, 90%.

The product was identified as

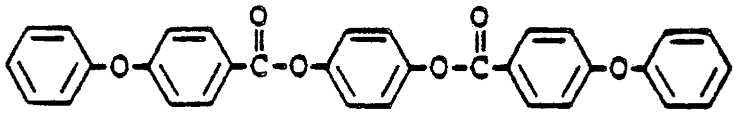

This monomer may be reacted with a mixture of terephthaloyl and isophthaloyl chlorides in 1,2-dichloroethane together with N,N-dimethylformamide and aluminium chloride to produce a polymer of the repeat unit:

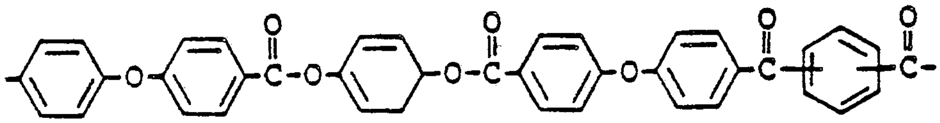

Example 3

To 125 mls of xylene was added 6.0 g of phenoxyphenol and 9.60 g of bis(tributyltin)oxide. The reaction mixture was heated to boiling and water of reaction removed using a Dean-Stark head. After all the water had been removed heating was discontinued and when refluxing had subsided 3.27 g of terephthaloyl chloride added. The mixture was heated to reflux for 3 hours and then allowed to cool. The colourless crystalline product was collected by filtration, washed with cold toluene and dried. A further crop of crystals was obtained by concentrating the mother liquor.

Yield was 7.5 g, 91%.

The product was identified as

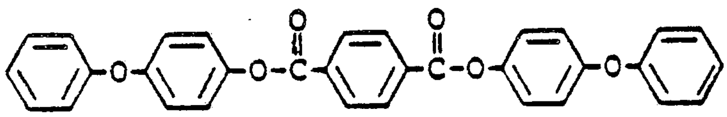

The analogous isophthaloyl product was prepared by repeating the above reaction, replacing the terephthaloyl dichloride with isophthaloyl dichloride.

This monomer may be repeated with amixture of terephthaloyl and isophthaloyl chloride in 1,2-dichloroethane together with N,N-dimethylformamide and aluminium chloride to produce a polymer of the repeat unit:

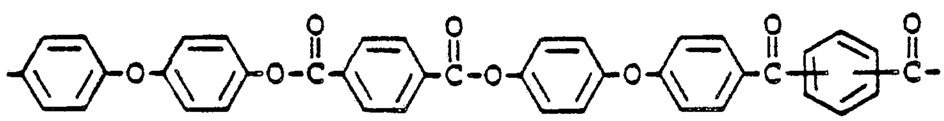

Example 4

To 500 mls of toluene was added 15.32 g of bisphenol A and 43.1 g of tributyltinmethoxide. The reaction was heated to boiling and using a fractionating column methanol was removed. When all the methanol had been removed heating was discontinued and when refluxing had subsided 18.86 g of benzoylchloride added. The mixture was then heated to reflux for 3 hours and then allowed to cool. The colourless crystalline product was collected by filtration, washed with cold toluene and dried. A further crop of crystals was obtained by concentrating the mother liquors.

Yield 27.2 g, 93%.

The product was identified as

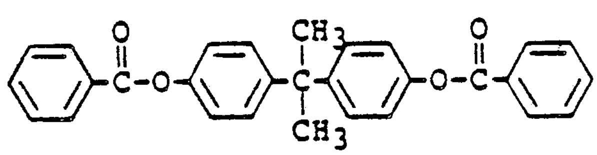

The above reaction was repeated a number of times, the bisphenol A being replaced in each repeated reaction with a different compound as indicated below:

a) replacing bisphenol A with hydroquinone to produce the monomer

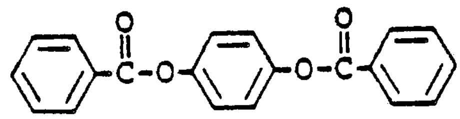

b) replacing bisphenol A with methylhydroquinone to produce the monomer

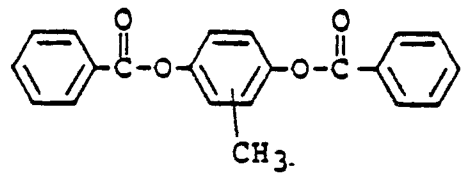

c) replacing bisphenol A with 4,4′-dihydroxydiphenyl ether to produce the monomer

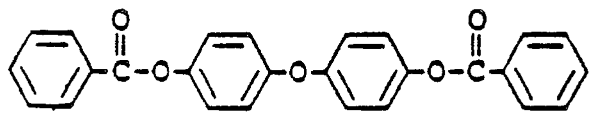

d) replacing bisphenol A with 4,4′-dihydroxybiphenyl to produce the monomer

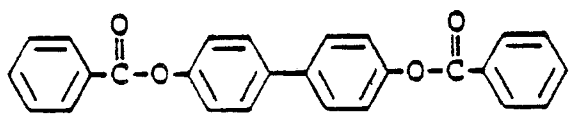

Example 5

To 100 mls of xylene was added 3.1 g of methylhydroquinone and 14.70 g of bis(tributyltin)oxide. The reaction mixture was heated to boiling and water of reaction removed using a Dean-Stark head. After all the water had been removed heating was discontinued and when refluxing had subsided 9 g of 4-n-propylbenzoyl chloride added. The mixture was then heated to reflux for 3 hours. The pale yellow solution was concentrated on a rotary evaporator leading a yellow oil. This was dissolved in methylcyclohexane and cooled to −15°C to give a colourless, crystalline product.

Yield 9 g, 86%.

The product was identified as

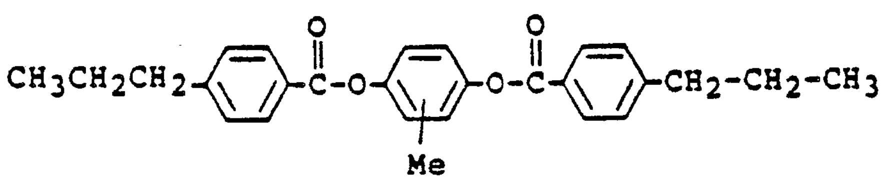

Example 6

To 200 mls of toluene was added 13.84 g of 4-bromophenol and 23.48 g of bistributyltinoxide. The reaction was heated to boiling and water of reaction removed using a Dean-Stark head. After all the water had been removed heating was discontinued and when refluxing had subsided 8.12 g of terephthaloyl chloride added. The mixture was then refluxed for two hours and then allowed to cool. The white crystalline product was collected by filtration, washed with cold toluene and dried. A further crop of crystals was obtained by concentrating the mother liquor.

Yield was 17.68 g, 93%.

The product was identified as

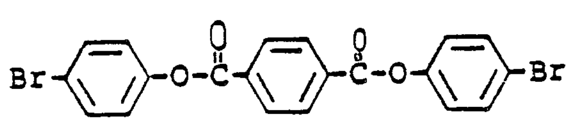

Example 7

To 150 mls of toluene was added 6.61 g of hydroquinone and 35.76 g of bis(tributyltin)oxide. The reaction mixture was heated to boiling and water of reaction removed using a Dean-Stark head. After all the water had been removed heating was discontinued and when refluxing had subsided 19.03 g of p-fluorobenzoyl chloride added. The mixture was then refluxed for one hours and then allowed to cool. The white crystalline product was collected by filtration, washed with cold toluene and dried. A further crop of crystals was obtained by concentrating the mother liquor.

Yield was 19.89 g, 93.5%.

The product was identified as

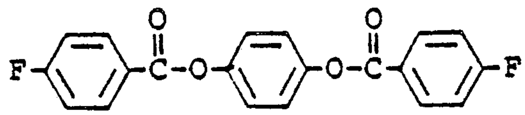

Example 8

To 150 mls of toluene was added 9.97 g of ethyl-4-hydroxybenzoate and 17.88 g of bis(tributyltin)oxide. The reaction mixture was heated to boiling and the water of reaction removed using a Dean-Stark head. After all the water had been removed heating was discontinued and when refluxing had subsided 27.92 g of 4-phenoxybenzoyl chloride added. The mixture was then refluxed for one hour and then allowed to cool. No precipitate formed on cooling. The toluene was removed under reduced pressure and replaced by hexane. After cooling to −18°C a white crystalline product was obtained. A further crop of crystals was obtained by concentrating the mother liquor. This material was very soluble at room temperature in a number of solvents.

Yield was 37.40 g, 86%.

The product was identified as

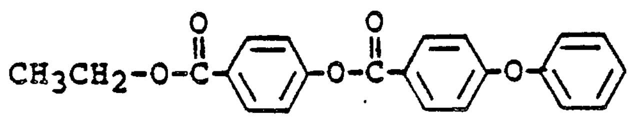

Example 9

To 150 mls of toluene was added 5.51 g of hydroquinone and 29.80 g of bis(tributyltin)oxide. The reaction mixture was heated to boiling and the water of reaction removed using a Dean-Stark head. After allowing to cool the solution was transferred to a dropping funnel equipped with a pressure equalising side arm. This solution was then slowly added to an almost refluxing solution of 30.45 g of terephthaloyl chloride in 150 mls of toluene. After the addition was complete the mixture was refluxed for 2 hours and then allowed to cool. The white crystalline product was collected by filtration washed with cold toluene and dried.

Yield 20 g, 88%.

The product was identified as

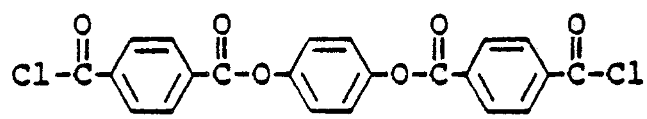

Example 10

To 200 ml of toluene was added 20.43 g of 4-phenylphenol and 35.76 g of bis(tributyltin)oxide. The reaction mixture was heated to boiling and the water of reaction removed using a Dean-Stark head. After all the water had been removed the reaction mixture was allowed to cool. When cool the solution was transferred to a dropping funnel fitted with a pressure equalising side arm. This solution was then slowly added to a warm solution of 48.72 g of terephthaloyl chloride and 150 mls of toluene. After the addition was complete the mixture was heated to reflux for one hour and then allowed to cool. The pale yellow crystalline product was collected by filtration washed with cold toluene and dried.

Two products were identified:

1) 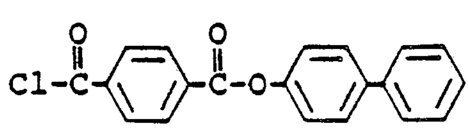 34.29g, 85% yield.

2) 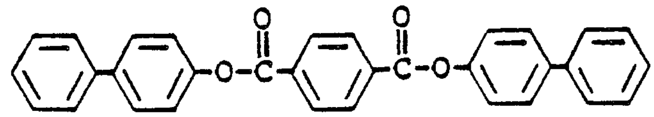 10% yield.

The product

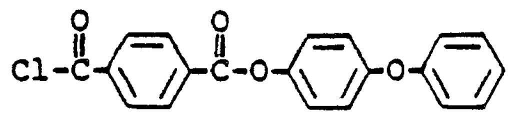

was prepared in a similar manner.

Example 11

To 200 mls of toluene was added 16.52 g of hydroquinone and 89.41 g of bis(tributyltin)oxide. The reaction mixture was heated to boiling and the water of reaction removed using a Dean-Stark head. After all the water had been removed heating was discontinued. Once the temperature of the solution has dropped to 80°C a solution of 12.18 g of terephthaloyl chloride in 50 mls of toluene was slowly added over 45 minutes. After the addition was complete the whole mixture was refluxed for 2 hours. Once cool the toluene was removed and replaced with 200 mls of methanol plus 20 mls of a 50% solution of a hydrochloric acid. The white solid was collected by filtration and washed with 300 mls of methanol at 50°C.

Yield 16.40 g, 80%.

The product was identified as

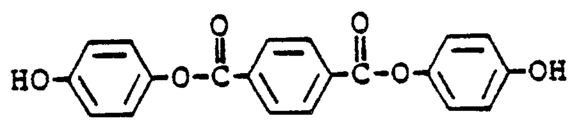

Example 12

To 150 mls of toluene was added 10 g of 4-phenoxyphenol and 16.00 g of bis(tributyltin)oxide. The reaction mixture was heated to boiling and the water of reaction removed using a Dean-Stark head. After all the water had been removed heating was discontinued and the mixture allowed to cool to room temperature. Once cool a solution of 2.66 g of phosgene in 18 mls. of toluene was slowly added. Upon completion of the addition the mixture was stirred at room temperature for 1 hour and then at reflux for 1 hour. After cooling to −18°C the white crystalline solid was collected by filtration washed with cold toluene and dried.

Yield 9.6 g, 89%.

The product was identified as

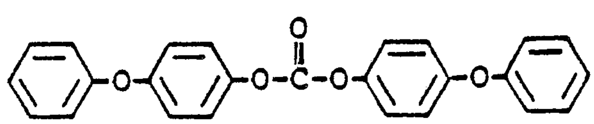

Example 13

To 200 mls of toluene was added 20 g of bisphenol A and 21.80 g of dibutyltin oxide. The reaction mixture was heated to boiling and water of reaction removed using a Dean-Stark head. After all the water had been removed heating was discontinued and the viscous solution allowed to cool to 90°C. To this solution was added 40.73 g of 4-phenoxybenzoyl chloride and the mixture heated to reflux for 1 hour. After cooling the white crystalline product was collected by filtration, washed with cold toluene and dried.

Yield 49.71 g, 91.5.

The product was identified as

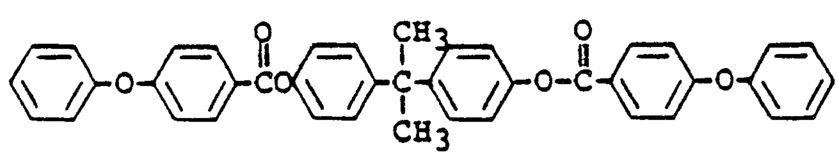

## Claims

1. A method for the preparation of an organic compound comprising reacting a first compound of the formula:

$$(R)_r\text{—M—Y—}A^1\text{—Y—M—}(R)_r$$

where

each R is independently a substituted or unsubstituted alkyl or aryl group;

each r is independently an integer from 1 to 4 inclusive depending upon the element M used;

each M is independently an element selected from Group IIIB, IVB or VB of the Periodic Table or a transition metal, excluding carbon, silicon, nitrogen, phosphorus, boron, aluminium and titanium;

each Y is independently an oxygen atom, a sulphur atom, a substituted nitrogen atom other than

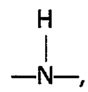,

or a substituted phoshorous atom other than

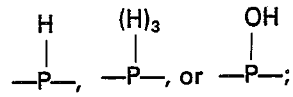

, , or ;

and $A^1$ is at least partly aromatic with each atom Y bonded to an aromatic group of $A^1$, with a second compound of the formula:

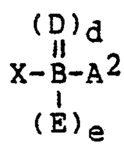

where

X is a halogen atom or a group capable of reacting with the first compound to eliminate a compound containing M and X;

B is an atom selected from carbon, phosphorus, sulphur or silicon;

D is an oxygen or sulphur atom or an amine group;

d is 1 if B is carbon, zero or 1 if B is phosphorus, zero, 1 or 2 if B is sulphur or zero if B is silicon;

E is selected from an aromatic group, aliphatic group, OR′ or $NR'_2$ if B is phosphorus, or from an aromatic group, aliphatic group or —OR′ if B is silicon, where R′ is a substituted or unsubstituted alkyl or aryl group;

e is zero if B is carbon or sulphur, 1 if B is phosphorus or 2 if B is silicon; and

$A^2$ is an aromatic, aliphatic, aromatic/aliphatic, heterocyclic, alicyclic, siloxyl or silane group,

to eliminate the compound $(R)_rMX$ and produce a compound of the formula:

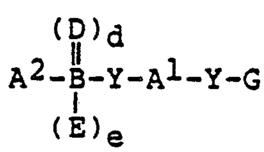

where G is either the group $\text{—M—}(R)_r$, or the group

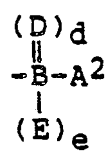

2. A method according to claim 1, wherein the stoichiometric ratio of the first compound to the second compound is 1:2 and reaction of the first and second compounds produces a compound of the formula:

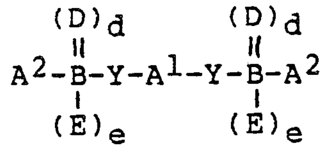

3. A method for the preparation of an organic compound comprising reacting a first compound of the formula:

$$(R)_r—M—Y—A^1—Y—M—(R)_r$$

where

each R is independently a substituted or unsubstituted alkyl or aryl group;

each r is independently an integer from 1 to 4 inclusive depending upon the element M used;

each M is independently an element selected from Group IIIB, IVB or VB of the Periodic Table (IUPAC 1965 revision) or a transition metal, excluding carbon, silicon, nitrogen, phosphorus, boron, aluminium and titanium;

each Y is independently an oxygen atom, a sulphur atom, a substituted nitrogen atom other than

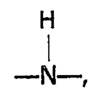

or a substituted phoshorous atom other than

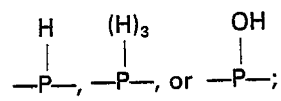

and $A^1$ is at least partly aromatic with each atom Y bonded to an aromatic group of $A^1$, with a second compound of the formula:

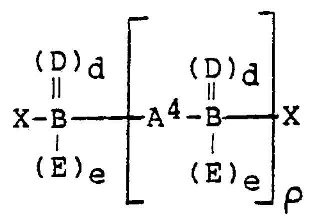

where

X is a halogen atom or a group capable of reacting with the first compound to eliminate a compound containing M and X;

$A^4$ is an aromatic, aliphatic, aromatic/aliphatic, heterocyclic, alicyclic, siloxyl or silane group,

B is an atom selected from carbon, phosphorus, sulphur or silicon;

D is an oxygen or sulphur atom or an amine group;

d is 1 if B is carbon, zero or 1 if B is phosphorus, zero, 1 or 2 if B is sulphur or zero if B is silicon;

E is selected from an aromatic group, aliphatic group, OR′ or $NR'_2$ if B is phosphorus, or from an aromatic group, aliphatic group or —OR′ if B is silicon, where R′ is a substituted or unsubstituted alkyl or aryl group;

e is zero if B is carbon or sulphur, 1 if B is phosphorus or 2 if B is silicon; and

p is zero or 1,

to eliminate the compound $(R)_rMX$ and produce a compound of the formula:

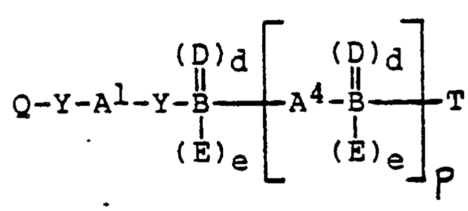

where Q is the either the group $(R)_rM—$, or the group

group 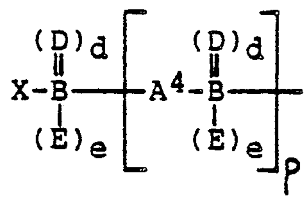 ;and

T is either the atom or group X, or the group —Y—$A^1$—Y—M—$(R)_r$.

4. A method according to claim 3 wherein the first and second compounds are reacted together by gradually adding a solution of the first compound to a solution of the second compound.

5. A method according to claim 3 or 4 wherein the first and second compounds are reacted together in a solvent in which the compound produced by the reaction is substantially insoluble so that the compound is precipitated substantially as soon as it is produced.

6. A method according to any one of claims 3 to 5 wherein p is 1.

7. A method according to claim 6 wherein the first and second compounds are reacted to produce a compound of the formula:

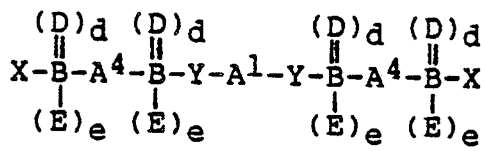

8. A method for the preparation of an organic compound comprising reacting a first compound of the formula:

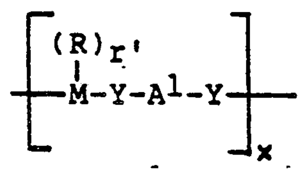

where

R is independently a substituted or unsubstituted alkyl or aryl group;

r' is independently zero or an integer from 1 to 3 depending upon the element M used;

each Y is independently an oxygen atom, a sulphur atom, a substituted nitrogen atom other than

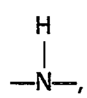,

or a substituted phoshorous atom other than

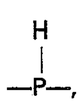, 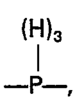, or 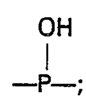;

$A^1$ is at least partly aromatic with each atom Y bonded to an aromatic group of $A^1$,

x is an integer greater than 1,

with a second compound of the formula:

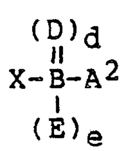

where

X is a halogen atom or a group capable of reacting with the first compound to eliminate a compound containing M and X;

B is an atom selected from carbon, phosphorus, sulphur or silicon;

D is an oxygen or sulphur atom or an amine group;

d is 1 if B is carbon, zero or 1 if B is phosphorus, zero, 1 or 2 if B is sulphur or zero if B is silicon;

E is selected from an aromatic group, aliphatic group, OR' or $NR'_2$ if B is phosphorus, or from an

aromatic group, aliphatic group or —OR′ if B is silicon, where R′ is a substituted or unsubstituted alkyl or aryl group;

e is zero if B is carbon or sulphur, 1 if B is phosphorus or 2 if B is silicon; and

$A^2$ is an aromatic, aliphatic, aromatic/aliphatic, heterocyclic, alicyclic, siloxyl or silane group, to eliminate the compound

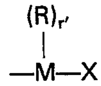

and produce a compound of the formula:

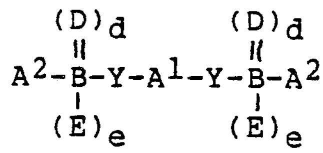

9. A method according to any one of the preceding claims wherein the group

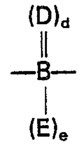

is a carbonyl group

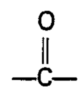

or a thiocarbonyl group

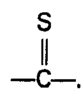

10. A method according to claim 9 wherein the group

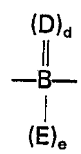

is a carbonyl group

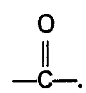

11. A method according to any one of the preceding claims wherein Y is oxygen or sulphur.

12. A method according to claim 11 wherein Y is oxygen.

13. A method according to any one of the preceding claims wherein the first and second compounds are selected so that reaction of the compounds produces an aromatic ester, aromatic thioester, aromatic carbonate or aromatic thiocarbonate compound.

14. A method for the preparation of an aromatic ester, aromatic thioester, aromatic carbonate or aromatic thiocarbonate compound comprising reacting a first compound of the formula:

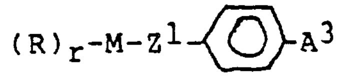

where

each R is independently a substituted or unsubstituted alkyl or aryl group;

r is an integer from 1 to 4 inclusive depending upon the element M used;

M is an element selected from Group IIIB, IVB or VB of the Periodic Table or a transition metal, excluding carbon, silicon, nitrogen, phosphorus, boron, aluminium and titanium;

$Z^1$ is an oxygen or a sulfur atom, and;

$A^3$ is an aromatic, aliphatic, aromatic/aliphatic, heterocyclic, alicyclic, siloxyl or silane group, or a hydrogen atom

with a second compound of the formula:

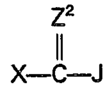

where

X is a halogen atom or a group capable of reacting with the first compound to eliminate a compound containing M and X;

$Z^2$ is either an oxygen or a sulphur atom; and

J is either the atom or group X, or the group $A^2$ where $A^2$ is an aromatic, aromatic/aliphatic, heterocyclic, alicyclic, siloxyl or silane group, or the group

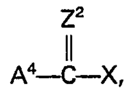

where $A^4$ is an aromatic, aliphatic, aromatic/aliphatic, heterocyclic, alicyclic, siloxyl or silane group, to eliminate thee compound $(R)_rMX$ and produce an aromatic ester, aromatic thioester, aromatic carbonate or aromatic thiocarbonate compound of the formula:

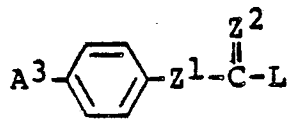

where L is either the atom or group X, the group $A^2$, the group

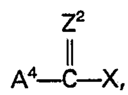

or the group

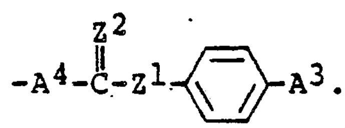

15. A method according to claim 14 wherein $Z^1$ is an oxygen atom.

16. A method according to claim 14 or 15 wherein $Z^2$ is an oxygen atom.

17. A method according to any one of claims 14 to 16 where the stoichiometric ratio of the first compound to the second compound is 1:1.

18. A method according to claim 17 wherein the second compound is of the formula:

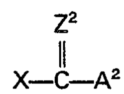

and the first and second compounds react to produce an aromatic ester, aromatic thioester, aromatic carbonate or aromatic thiocarbonate compound of the formula:

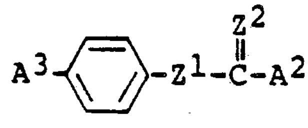

19. A method according to claim 17 wherein the second compound is of the formula:

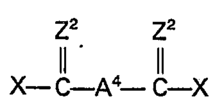

and the first and second compounds react to produce an aromatic ester, aromatic thioester, aromatic carbonate or aromatic thiocarbonate of the formula:

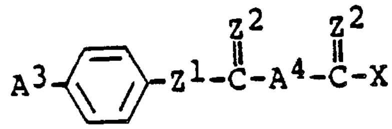

20. A method according to claim 19 wherein the first and second compounds are reacted together by gradually adding a solution of the first compound to a solution of the second compound.

21. A method according to claim 19 or 20 wherein the first and second compounds are reacted together in a solvent in which the compound produced by the reaction is substantially insoluble so that the compound is precipicated substantially as soon as it is produced.

22. A method according to any one of the claims 14 to 16 wherein the second compound is of the formula:

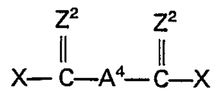

and the first and second compounds are reacted together in a stoichiometric ratio of 2:1 to produce an aromatic ester, aromatic thioester, aromatic carbonate or aromatic thiocarbonate of the formula:

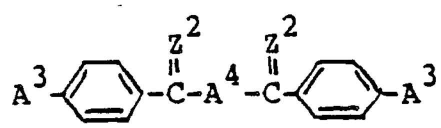 .

23. A method according to any preceding claim wherein each group $A^2$ or $A^4$ is at least partly aromatic and the groups —B— or

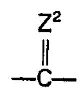

are each bonded to an aromatic group of $A^2$ or $A^4$.

24. A method according to any one of the preceding claims wherein M is selected from Group IVB of the Periodic Table.

25. A method according to claim 24 wherein M is tin.

26. A method according to claim 23 or 24 wherein r is 3.

27. A method according to any one of the preceding claims wherein R is a butyl group.

28. A method according to any one of the preceding claims wherein X is a halogen atom.

29. A method according to claim 30 wherein X is a chlorine atom.

30. A method according to any one of the preceding claims which produces an organic compound of the formula:

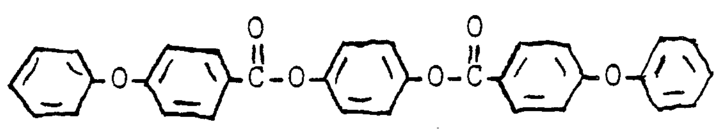 or

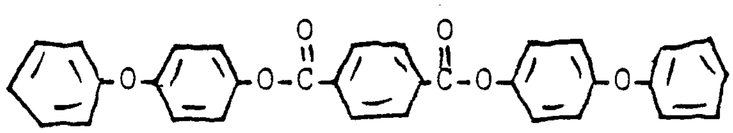 or

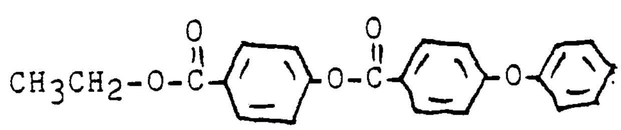 or

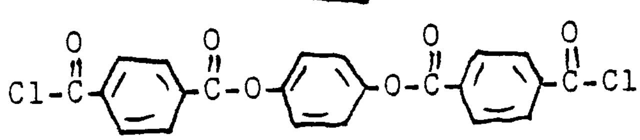 or

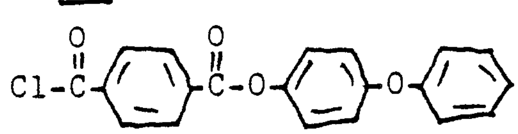

* * * * * *

**Patentansprüche**

1. Verfahren zur Herstellung einer organischen Verbindung, bestehend daraus, daß man eine erste Verbindung der Formel:

$$(R)_r—M—Y—A^1—Y—M—(R)_r$$

worin

jedes R unabhängig eine substituierte oder unsubstituierte Alkyl- oder Arylgruppe ist;

jedes r unabhängig eine ganze Zahl von 1 bis einschließlich 4, abhängig von dem verwendeten Element M bedeutet;

jedes M unabhängig ein Element ist, ausgewählt aus den Gruppen IIIB, IVB oder VB des Periodensystems oder einem Übergangsmetall, ausgenommen Kohlenstoff, Silizium, Stickstoff, Phosphor, Bor, Aluminium und Titan;

jedes Y unabhängig ein Sauerstoffatom, ein Schwefelatom, ein substituiertes Stickstoffatom außer

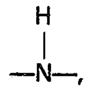

oder ein substituiertes Phosphoratom außer

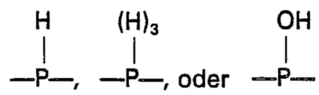

ist und

$A^1$ mindestens teilweise aromatisch ist, wobei jedes Atom Y an eine aromatische Gruppe von $A^1$ gebunden ist,

mit einer zweiten Verbindung der nachstehenden Formel reagieren läßt:

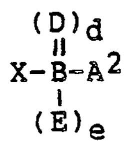

worin

X ein Halogenatom oder eine Gruppe ist, die mit der ersten Verbindung reagieren kann zur Eliminierung einer Verbindung, die M und X enthält;

B ein Atom ist, ausgewählt aus Kohlenstoff, Phosphor, Schwefel oder Silizium;

D ein Sauerstoff- oder Schwefelatom oder eine Amingruppe ist;

d 1 ist, wenn B Kohlenstoff ist, 0 oder 1 ist, wenn B Phosphor ist, 0, 1 oder 2 ist, wenn B Schwefel ist, oder 0 ist, wenn B Silizium ist;

E ausgewählt ist aus einer aromatischen Gruppe, einer aliphatischen Gruppe, OR' oder $NR'_2$, wenn B Phosphor ist oder aus einer aromatischen Gruppe, einer aliphatischen Gruppe oder —OR', wenn B Silizium ist, wobei R' eine substituierte oder unsubstituierte Alkyl- oder Arylgruppe ist;

e 0 ist, wenn B Kohlenstoff oder Schwefel itst, 1 ist, wenn B Phosphor ist, oder 2 ist, wenn B Silizium ist; und

$A^2$ eine aromatische, aliphatische, aromatisch/aliphatische, heterocyclische, alicyclische, Siloxyl- oder Silangruppe ist, um die Verbindung $(R)_rMX$ zu eliminieren und eine Verbindung der Formel:

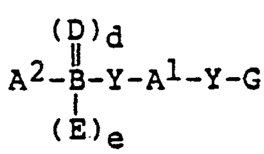

herzustellen, worin G entweder die Gruppe $—M—(R)_r$ oder die Gruppe

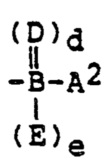

bedeutet.

2. Verfahren nach Anspruch 1, worin das stöchiometrische Verhältnis der ersten Verbindung zur zweiten Verbindung 1:2 ist und die Reaktion der ersten und zweiten Verbindungen eine Verbindung der Formel:

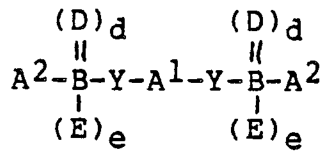

liefert.

3. Verfahren zur Herstellung einer organischen Verbindung, worin man eine erste Verbindung der Formel:

$$(R)_r—M—Y—A^1—Y—M—(R)_r$$

worin

jedes R unabhängig eine substituierte oder unsubstituierte Alkyl- oder Arylgruppe bedeutet;

jedes r unabhängig eine ganze Zahl von 1 bis einschließlich 4 abhängig von dem verwendeten Element M bedeutet;

jedes M unabhängig ein Element ist, ausgewählt aus den Gruppen IIIB, IVB oder VB des Periodensystems (UIPAC 1965 Revision) oder einem Übergangsmetall, ausgenommen Kohlenstoff, Silizium, Stickstoff, Phosphor, Bor, Aluminium und Titan;

jedes Y unabhängig ein Sauerstoffatom, ein Schwefelatom, ein substituiertes Stickstoffatom außer

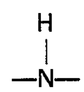

oder ein substituiertes Phosphoratom außer

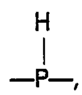, 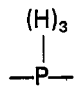 oder 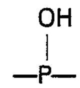

bedeutet und

$A^1$ mindestens teilweise aromatisch ist, wobei jedes Atom Y an eine aromatische Gruppe von $A^1$ gebunden ist, mit einer zweiten Verbindung der Formel:

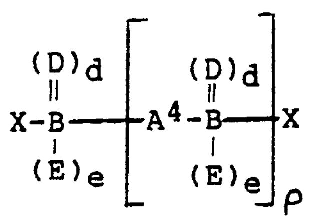

umsetzt, worin

X ein Halogenatom oder eine Gruppe, die mit der ersten Verbindung zur Eliminierung einer M und X enthaltenden Verbindung reagieren kann, bedeutet;

$A^4$ eine aromatische, aliphatische, aromatisch/aliphatische, heterocyclische, alicyclische, Siloxyl- oder Silangruppe bedeutet,

B ein Atom ist, ausgewählt aus Kohlenstoff, Phosphor, Schwefel oder Silizium;

D ein Sauerstoff- oder Schwefelatom oder eine Amingruppe ist;

d 1 ist, wenn B Kohlenstoff ist, 0 oder 1 ist, wenn B Phosphor ist, 0, 1 oder 2 ist, wenn B Schwefel ist, oder 0 ist, wenn B Silizium ist;

E ausgewählt ist aus einer aromatischen Gruppe, einer aliphatischen Gruppe, OR′ oder $NR'_2$, wenn B Phosphor ist, oder aus einer aromatischen Gruppe, einer aliphatischen Gruppe oder —OR′, wenn B Silizium ist, wobei R′ eine substituierte oder unsubstituierte Alkyl- oder Arylgruppe ist;

e 0 ist, wenn B Kohlenstoff oder Schwefel ist, 1 ist, wenn B Phosphor ist, oder 2 ist, wenn B Silizium ist; und

p 0 oder 1 ist, um die Verbindung $(R)_rMX$ zu eliminieren und eine Verbindung der Formel:

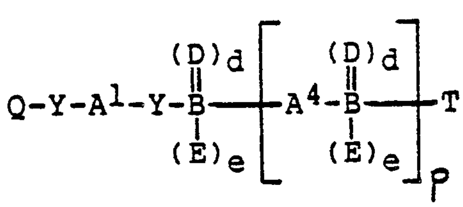

worin Q entweder die Gruppe $(R)_rM$- oder die Gruppe

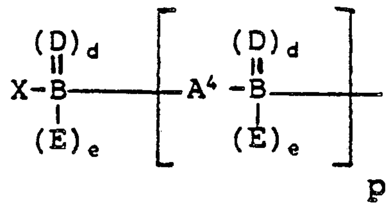 bedeutet und

T entweder das Atom oder die Gruppe X oder die Gruppe $Y—A^1—Y—M—(R)_r$ ist, herzustellen.

4. Verfahren nach Anspruch 3, worin die ersten und zweiten Verbindungen miteinander umgesetzt werden, indem allmählich eine Lösung der ersten Verbindung zu einer Lösung der zweiten Verbindung zugegeben wird.

5. Verfahren nach Anspruch 3 oder 4, worin die ersten und zweiten Verbindungen miteinander umgesetzt werden in einem Lösungsmittel, in dem die durch die Reaktion hergestellte Verbindung im wesentlichen unlöslich ist, so daß die Verbindung im wesentlichen, sobald sie entsteht, ausfällt.

6. Verfahren nach einem der Ansprüche 3 bis 5, worin p gleich 1 ist.

7. Verfahren nach Anspruch 6, worin die ersten und zweiten Verbindungen umgesetzt werden, um eine Verbindung der Formel:

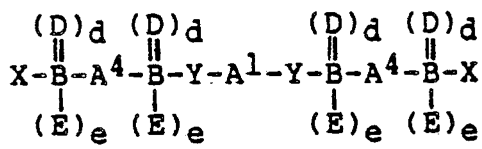

herzustellen.

8. Verfahren zur Herstellung einer organischen Verbindung, bestehend daraus, daß man eine erste Verbindung der Formel:

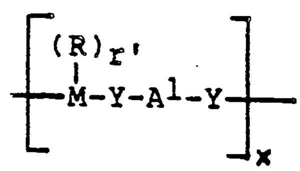

worin

R unabhängig eine substituierte oder unsubstituierte Alkyl- oder Arylgruppe bedeutet;

r' unabhängig 0 oder eine ganze Zahl von 1 bis 3, abhängig vom verwendeten Element M beduetet;

jedes Y unabhängig ein Sauerstoffatom, ein Schwefelatom, ein substituiertes Stickstoffatom außer

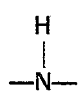

oder ein substituiertes Phosphoratom außer

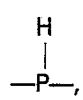, 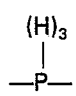 oder 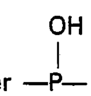

bedeutet;

$A^1$ mindestens teilweise aromatisch ist, wobei jedes Atom Y an eine aromatische Gruppe von $A^1$ gebunden ist,

x eine ganze Zahl größer als 1 ist, mit einer zweiten Verbindung der Formel umsetzt:

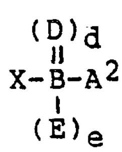

worin

X ein Halogenatom oder eine Gruppe, die mit der ersten Verbindung zur Eliminierung einer M und X enthaltenden Verbindung reagieren kann, ist;

B ein Atom ist, ausgewählt aus Kohlenstoff, Phosphor, Schwefel oder Silizium;

D ein Sauerstoff- oder Schwefelatom oder eine Amingruppe ist;

d 1 ist, wenn B Kohlenstoff ist, 0 oder 1 ist, wenn B Phosphor ist, 0, 1 oder 2 ist, wenn B Schwefel ist, oder 0 ist, wenn B Silizium ist;

E ausgewählt ist aus einer aromatischen Gruppe, einer aliphatischen Gruppe, OR' oder $NR'_2$, wenn B Phosphor ist oder aus einer aromatischen Gruppe, einer aliphatischen Gruppe oder —OR', wenn B Silizium ist, wobei R' eine substituierte oder unsubstituierte Alkyl- oder Arylgruppe ist;

e 0 ist, wenn B Kohlenstoff oder Schwefel ist, 1 ist, wenn B Phosphor ist, oder 2 ist, wenn B Silizium ist; und

$A^2$ eine aromatische, aliphatische, aromatisch/aliphatische, heterocyclische, alicyclische, Siloxyl- oder Silangruppe ist, um die Verbindung

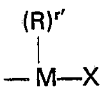

zu eliminieren und eine Verbindung der Formel:

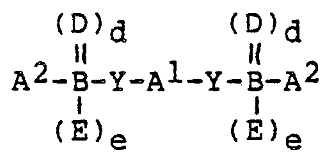

herzustellen.

9. Verfahren nach einem der vorhergehenden Ansprüche, worin, die Gruppe

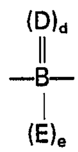

eine Carbonylgruppe

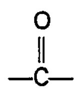 oder

ein Thiocarbonylgruppe

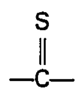

ist.

10. Verfahren nach Anspruch 9, worin die Gruppe

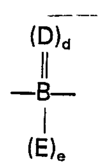

eine Carbonylgruppe

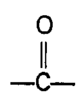

ist.

11. Verfahren nach einem der vorhergehenden Ansprüche, worin Y Sauerstoff oder Schwefel ist.

12. Verfahren nach Anspruch 11, worin Y Sauerstoff ist.

13. Verfahren nach einem der vorhergehenden Ansprüche, worin die ersten und zweiten Verbindungen so ausgewählt sind, daß die Reaktion der Verbindungen einen aromatischen Ester, aromatischen Thioester, ein aromatisches Carbonat oder aromatisches Thiocarbonat liefert.

14. Verfahren zur Herstellung eines aromatischen Esters, aromatischen Thioesters, aromatischen Carbonats oder aromatischen Thiocarbonats, bestehend daraus, daß man eine erste Verbindung der Formel:

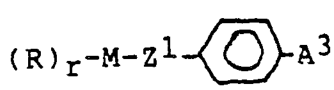

worin jedes

R unabhängig eine substituierte oder unsubstituierte Alkyl- oder Arylgruppe bedeutet;

r eine ganze Zahl von 1 bis einschließlich 4, abhängig von dem verwendeten Element M ist;

M ein Element ist, ausgewählt aus Gruppe IIIB, IVB oder VB des Periodensystems, oder ein Übergangsmetall, ausgenommen Kohlenstoff, Silizium, Stickstoff, Phosphor, Aluminium und Titan;

$Z^1$ ein Sauerstoff- oder Schwefelatom ist; und

$A^3$ eine aromatische, aliphatische, aromatisch/aliphatische, heterocyclische, alicyclische, Siloxyl- oder Silangruppe oder ein Wasserstoffatom ist,

mit einer zweiten Verbindung der Formel umsetzt:

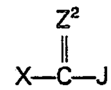

worin

X ein Halogenatom oder eine Gruppe ist, die mit der ersten Verbindung zur Eliminierung vom M und X reagieren kann;

$Z^2$ entweder ein Sauerstoff- oder ein Schwefelatom ist; und

J entweder das Atom oder die Gruppe X oder die Gruppe $A^2$ ist, worin $A^2$ eine aromatische, aliphatische, aromatisch/aliphatische, heterocyclische, alicyclische, Siloxyl- oder Silangruppe ist, oder die Gruppe

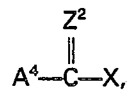

worin $A^4$ eine aromatische, aliphatische, aromatisch/aliphatische, heterocyclische, alicyclische, Siloxyl- oder Silangruppe bedeutet, um die Verbindung $(R)_rMX$ zu eliminieren und einen aromatischen Ester, aromatischen Thioester, ein aromatisches Carbonat oder aromatisches Thiocarbonat der Formel:

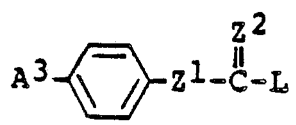

herzustellen, worin L entweder das Atom oder die Gruppe X, die Gruppe $A^2$, die Gruppe

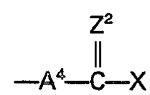

oder die Gruppe

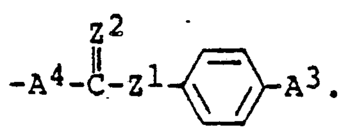

bedeutet.

15. Verfahren nach Anspruch 14, worin $Z^1$ ein Sauerstoffatom ist.

16. Verfahren nach Anspruch 14 oder 15, worin $Z^2$ ein Sauerstoffatom ist.

17. Verfahren nach einem der Ansprüche 14 bis 16, worin das stöchiometrische Verhältnis der ersten Verbindung zur zweiten Verbindung 1:1 ist.

18. Verfahren nach Anspruch 17, worin die zweite Verbindung die Formel:

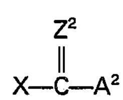

hat und die ersten und zweiten Verbindungen reagieren, wobei ein aromatischer Ester, aromatischer Thioester, aromatisches Carbonat oder aromatisches Thiocarbonat der Formel:

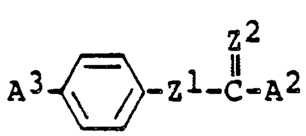

gebildet wird.

19. Verfahren nach Anspruch 17, worin die zweite Verbindung die Formel hat:

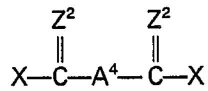

und die ersten und zweiten Verbindungen reagieren, wobei ein aromatischer Ester, aromatischer Thioester, ein aromatisches Carbonat oder aromatisches Thiocarbonat der Formel:

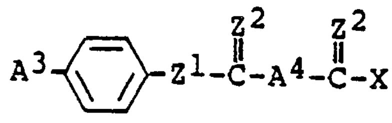

entsteht.

20. Verfahren nach Anspruch 19, worin die ersten und zweiten Verbindungen miteinander umgesetzt werden, in dem eine Lösung den ersten Verbindung allmählich zu einer Lösung der zweiten Verbindung zugegeben wird.

21. Verfahren nach Anspruch 19 oder 20, worin die ersten und zweiten Verbindungen miteinander umgesetzt werden in einem Lösungsmittel, in dem die durch die Reaktion hergestellte Verbindung im wesentlichen unlöslich ist, so daß die Verbindung im wesentlichen, sobald sie gebildet wird, ausfällt.

22. Verfarhen nach einem der Ansprüche 14 bis 16, worin die zweite Verbindung die Formel aufweist:

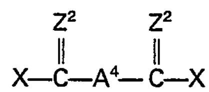

und die ersten und zweiten Verbindungen miteinander umgesetzt werden in einem stöchiometrischen Verhältnis von 2:1, um einen aromatischen Ester, aromatischen Thioester, ein aromatisches Carbonat oder aromatisches Thiocarbonat der Formel:

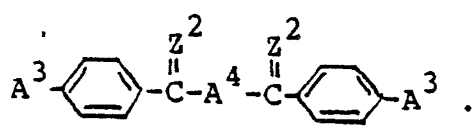

herzustellen.

23. Verfahren nach einem der vorhergehenden Ansprüche, worin jede Gruppe $A^2$ oder $A^4$ zumindest teilweise aromatisch ist und die Gruppen —B— oder

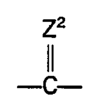

jeweils an eine aromatische Gruppe von $A^2$ oder A4 gebunden sind.

24. Verfahren nach einem der vorhergehenden Ansprüche, worin M ausgewählt ist aus der Gruppe IVB des Periodensystems.

25. Verfahren nach Anspruch 24, worin M Zinn ist.

26. Verfahren nach Anspruch 23 oder 24, worin r gleich 3 ist.

27. Verfahren nach einem der vorhergehenden Ansprüche, worin R eine Butylgruppe ist.

28. Verfahren nach einem der vorhergehenden Ansprüche, worin X ein Halogenatom ist.

29. Verfahren nach Anspruch 30, worin X ein Chloratom ist.

30. Verfahren nach einem der vorhergehenden Ansprüche, worin eine organische Verbindung der folgenden Formeln hergestellt wird:

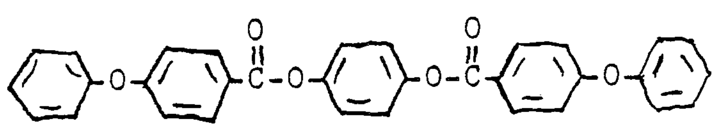 oder

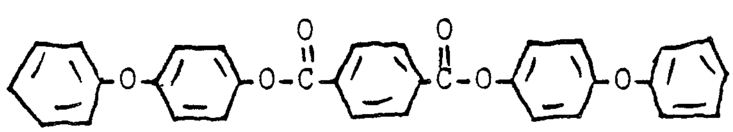 oder

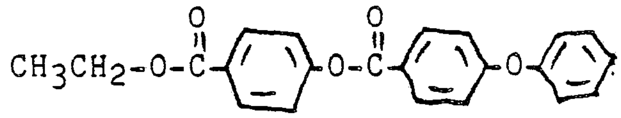 oder

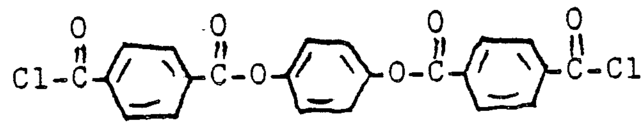 oder

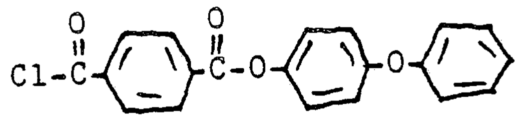

* * * * * *

**Revendications**

1. Procédé pour la préparation d'un composé organique comprenant la réaction d'un premier composé de formule:

$$(R)_r—M—Y—A^1—Y—M—(R)_r$$

dans laquelle

chaque R est indépendamment un groupe alkyle ou aryle substitué ou non substitué;

chaque r est indépendamment un nombre entier allant de 1 à 4 inclus, en fonction de l'élément M utilisé;

chaque M est indépendamment un élément choisi dans le groupe IIIB, IVB ou VB du tableau périodique (IUPAC, révision de 1965) ou un métal de transition, à l'exclusion du carbone, du silicium, de l'azote, du phosphore, du bore, de l'aluminium et du titane;

chaque Y est indépendamment un atome d'oxygène ou un atome de soufre, un atome d'azote substitué différent de

H
|
—N—,

ou un atome de phosphore substitué différent de

H     $(H)_3$     OH
|     |     |
—P—, —P—, ou —P—; et

$A^1$ est un radical au moins partiellement aromatique, chaque atome Y étant lié à un fragment aromatique de $A^1$,

avec un second composé de formule

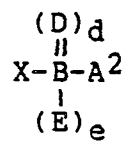

dans laquelle

X est un atome d'halogène ou un groupe capable de réagir avec le premier composé pour éliminer un composé contenant M et X;

B est un atome choisi parmi les atomes de carbone, phosphore, soufre et silicium;

D est un atome d'oxygène ou de soufre ou le groupe amino;

d est 1 lorsque B est un atome de carbone, zéro ou 1 lorsque B est un atome de phosphore, zéro, 1 ou 2 lorsque B est un atome de soufre, ou zéro lorsque B est un atome de silicium;

E est choisi parmi un groupe aromatique, un groupe aliphatique, OR' ou $NR'_2$ lorsque B est un atome de phosphore, ou parmi un groupe aromatique, un groupe aliphatique ou —OR' lorsque B est un atome de silicium, R' étant un radical alkyle ou aryle substitué ou non substitué;

e est zéro lorsque B est un atome de carbone ou de soufre, 1 lorsque B est un atome de phosphore ou 2 lorsque B est un atome de silicium; et

$A^2$ est un groupe aromatique, alphatique, aromatique/aliphatique, hétérocyclique, alicyclique, siloxy ou silane,

pour éliminer le composé $(R)_rMX$ et produire un composé de formule

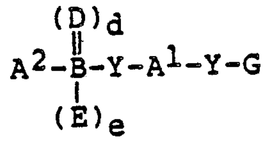

dans laquelle G est soit le groupe —M—$(R)_r$, soit le groupe

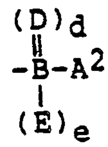

2. Procédé selon la revendication 1, dans lequel le rapport stoechiométrique du premier composé au second composé est 1:2 et la réaction du premier composé et du second donne un composé de formule:

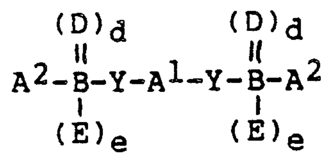

3. Procédé pour la préparation d'un composé organique, comprenant la réaction d'un premier composé de formule:

$$(R)_r—M—Y—A^1—Y—M—(R)_r$$

dans laquelle·

chaque R est indépendamment un groupe alkyle ou aryle substitué ou non substitué;

chaque r est indépendamment un nombre entier allant de 1 à 4 inclus, en fonction de l'élément M utilisé;

chaque M est indépendamment un élément choisi dans le groupe IIIB, IVB ou VB du tableau périodique (IUPAC, révision de 1965) ou un métal de transition, à l'exclusion du carbone, du silicium, de l'azote, du phosphore, du bore, de l'aluminium et du titane;

chaque Y est indépendamment un atome d'oxygène un atome de soufre, un atome d'azote substitué différent de

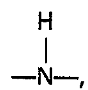,

ou un atome de phosphore substitué différent de

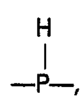, 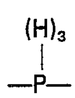 ou 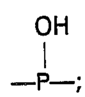; et

$A^1$ est un radical au moins partiellement aromatique, chaque atome Y étant lié à un fragment aromatique de $A^1$,

avec un second composé de formule

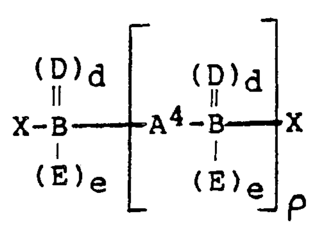

dans laquelle

X est un atome d'halogène ou un groupe capable de réagir avec le premier composé pour éliminer un composé contenant M et X;

$A^4$ est un groupe aromatique, aliphatique, aromatique/aliphatique, hétérocyclique, alicyclique, siloxy ou silane;

B est un atome choisi parmi les atomes de carbone, phosphore, soufe et silicium;

D est un atome d'oxygène ou de soufre ou le groupe amino;

d est 1 lorsque B est un atome de carbone, zéro ou 1 lorsque B est un atome de phosphore, zéro, 1 ou 2 lorsque B est un atome de soufre, ou zéro lorsque B est un atome de silicium;

E est choisi parmi un groupe aromatique, un groupe aliphatique, OR' ou $NR'_2$ lorsque B est un atome de phosphore, ou parmi un groupe aromatique, un groupe aliphatique ou —OR' lorsque B est un atome de silicium, R' étant un radical alkyle ou aryle substitué ou non substitué;

e est zéro lorsque B est un atome de carbone ou de soufre, 1 lorsque B est un atome de phosphore ou 2 lorsque B est un atome de silicium; et

p est zéro ou 1,

pour éliminer le composé $(R)_rMX$ est produire un composé de formule:

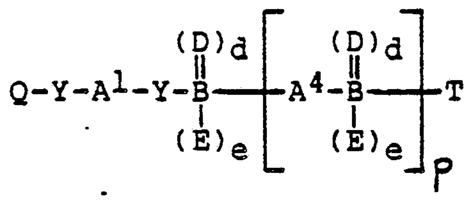

dans laquelle

Q est soit le groupe —M—$(R)_r$, soit le groupe

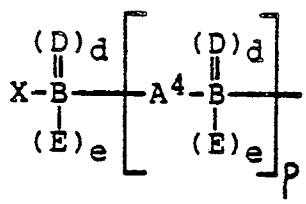 ; et

T est soit l'atome ou groupe X, soit le groupe —Y—$A^1$—Y—M—$(R)_r$.

4. Procédé selon la revendication 3, dans lequel on fait réagir ensemble le premier composé et le second par addition progressive d'une solution du premier composé à une solution du second composé.

5. Procédé selon la revendication 3 ou 4, dans lequel on fait réagir ensemble le premier composé et le second dans un solvant dans lequel le composé produit par la réaction est pratiquement insoluble, de sorte que le composé est précipité pratiquement dès sa formation.

6. Procédé selon l'une quelconque des revendications 3 à 5, dans lequel p est égal à 1.

7. Procédé selon la revendication 6, dans lequel on fait réagir le prmier composé et le second pour obtenir un composé de formule:

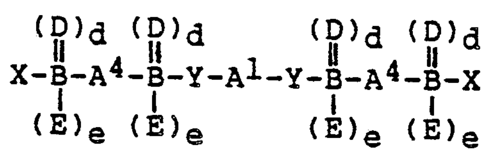

8. Procédé pour la préparation d'un composé organique, comprenant la réaction d'un premier composé de formule:

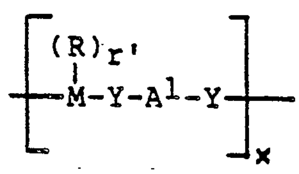

dans laquelle

R est indépendamment un groupe alkyle ou aryle substitué ou non substitué;

r' est indépendamment zéro ou un nombre entier allant de 1 à 3 en fonction de l'élément M utilisé;

chaque Y est indépendamment un atome d'oxygène, un atome de soufre, un atome d'azote substitué différent de

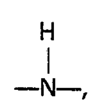

ou un atome de phosphore substitué différent de

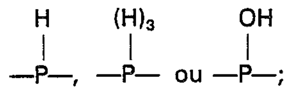

$A^1$ est un radical au moins partiellement aromatique, chaque atome Y étant lié à un fragment aromatique de $A^1$,

x est un nombre entier supérieur à 1, avec un second composé de formule:

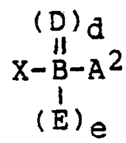

dans laquelle

X est un atome d'halogène ou un groupe capable de réagir avec le premier composé pour éliminer un composé contenant M et X;

B est un atome choisi parmi les atomes de carbone, phosphore, soufe et silicium;

D est un atome d'oxygène ou de soufre ou le groupe amino;

d est 1 lorsque B est un atome de carbone, zéro ou 1 lorsque B est un atome de phosphore, zéro, 1 ou 2 lorsque B est un atome de soufre, ou zéro lorsque B est un atome de silicium;

E est choisi parmi un groupe aromatique, un groupe aliphatique, OR' ou $NR'_2$ lorsque B est un atome de phosphore, ou parmi un groupe aromatique, un groupe aliphatique ou —OR' lorsque B est un atome de silicium, R' étant un radical alkyle ou aryle substitué ou non substitué;

e est zéro lorsque B est un atome de carbone ou de soufre, 1 lorsque B est un atome de phosphore ou 2 lorsque B est un atome de silicium; et

$A^2$ est un groupe aromatique, alphatique, aromatique/aliphatique, hétérocyclique, alicyclique, siloxy ou silane,

pour éliminer le composé

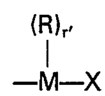

et produire un composé de formule:

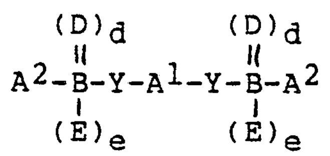

9. Procédé selon l'une quelconque des revendications precédéntes, dans lequel le groupe

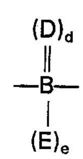

est le groupe carbonyle

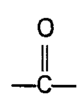

ou le groupe thiocarbonyle

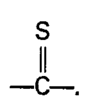

10. Procédé selon la revendication 9, dans lequel le groupe

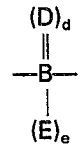

est le groupe carbonyle

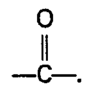

11. Procédé selon l'une quelconque des revendications précédentes, dans lequel Y est un atome d'oxygène ou de soufre.

12. Procédé selon la revendication 11, dans lequel Y est un atome d'oxygène.

13. Procédé selon l'une quelconque des revendications précédentes, dans lequel le premier composé et le second sont choisis de telle manière que la réaction des composés donne un composé de type ester aromatique, thioester aromatique, carbonate aromatique ou thiocarbonate aromatique.

14. Procédé pour la préparation d'un composé de type ester aromatique, thioester aromatique, carbonate aromatique ou thiocarbonate aromatique, comprenant la réaction d'un premier composé de formule:

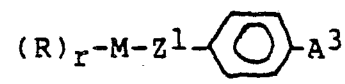

dans lequelle
chaque

R est indépendamment un group alkyle ou aryle substitué ou non substitué;

r est un nombre entier allant de 1 à 4inclusivement, en fonction de l'élément M utilisé;

M est un élément choisi dans le groupe IIIB, IVB ou VB du tableau périodique, ou un métal de transition à l'exclusion du carbone, du silicium, de l'azote, du phosphore, du bore, de l'aluminium et du titane;

$Z^1$ est un atome d'oxygène ou de soufre; et

$A^3$ est un groupe aromatique, aliphatique, aromatique/aliphatique, hétérocyclique, alicyclique, siloxy ou silane, ou un atome d'hydrogène,

avec un second composé de formule:

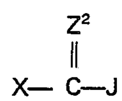

dans laquelle

X est un atome d'halogène ou un groupe capable de réagir avec le premier composé pour éliminer M et X;

$Z^2$ est soit un atome d'oxygène, soit un atome de soufre; et

J est soit l'atome ou groupe X, soit le groupe $A^2$, $A^2$ étant un groupe aromatique, alphatique, aromatique/aliphatique, hétérocyclique, alicyclique, siloxy ou silane, ou un radical

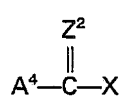

dans lequel $A^4$ est un groupe aromatique, aliphatique, aromatique/aliphatique, hétérocyclique, alicyclique, siloxy ou silane,

pour éliminer le composé $(R)_rMX$ et produire un composé de type ester aromatique, thioester aromatique, carbonate aromatique ou thiocarbonate aromatique, de formule:

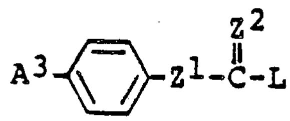

dans laquelle L est l'atome ou groupe X, le groupe $A^2$, le radical

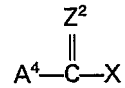

ou un groupe

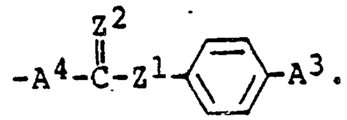

15. Procédé selon la revendication 14, dans lequel $Z^1$ est un atome d'oxygène.

16. Procédé selon la revendication 14 ou 15, dans lequel $Z^2$ est un atome d'oxygène.

17. Procédé selon l'une quelconque des revendications 14 à 16, dans lequel le rapport stoechiométrique du premier composé au second composé est 1:1.

18. Procédé selon la revendication 17, dans lequel le second composé est de formule

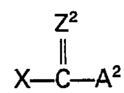

et le premier composé et le second réagissent pour donner un composé de type ester aromatique, thioester aromatique, carbonate aromatique ou thiocarbonate aromatique, de formule:

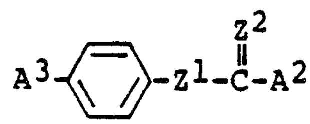

19. Procédé selon la revendication 17, dans lequel le second composé est de formule:

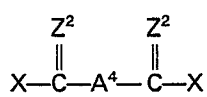

et le premiér composé et le second réagissent pour former un ester aromatique, thioester aromatique, carbonate aromatique ou thiocarbonate aromatique, de formule:

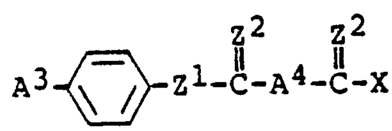

20. Procédé selon la revendication 19, dans lequel on fait réagir ensemble le premier composé et le second par addition progressive d'une solution du premier composé à une solution du second composé.

21. Procédé selon la revendication 19 ou 20, dans lequel on fait réagir ensemble le premier composé et le second dans un solvant dans lequel le composé produit par la réaction est pratiquement insoluble, de sorte que le composé est précipité pratiquement dès sa formation.

22. Procédé selon l'une quelconque des revendications 14 à 16, dans lequel le second composé est de formule:

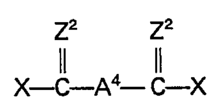

et on fait réagir ensemble le premier composé et le second en le rapport stoechiométrique 2:1 pour obtenir un ester aromatique, thioester aromatique, carbonate aromatique ou thiocarbonate aromatique, de formule

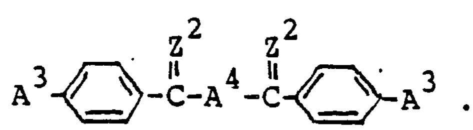

23. Procédé selon l'une quelconque des revendications précédentes, dans lequel chaque groupe $A^2$ ou $A^4$ est au moins partiellement aromatique et les groupes —B— ou

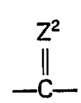

sont liés chacun à un groupe aromatique de $A^2$ ou $A^4$.

24. Procécé selon l'une quelconque des revendications précédentes, dans lequel M est choisi dans le groupe IVB du tableau périodique.

25. Procédé selon la revendication 24, dans lequel M est l'étain.

26. Procédé selon la revendication 23 ou 24, dans lequel r est égal à 3.

27. Procédé selon l'une quelconque des revendications précédentes, dans lequel R est le groupe butyle.

28. Procédé selon l'une quelconque des revendications précédentes, dans lequel X est un atome d'halogène.

29. Procédé selon la revendication 30, dans lequel X est un atome de chlore.

30. Procédé selon l'une quelconque des revendications précédentes, lequel donne un composé organique de formule:

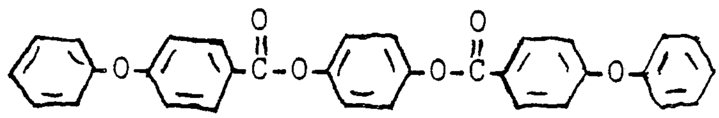

ou

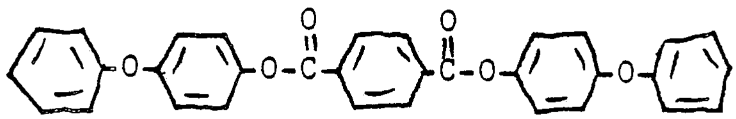

ou

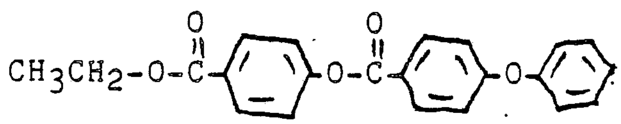

ou

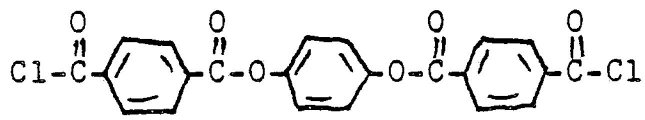

ou

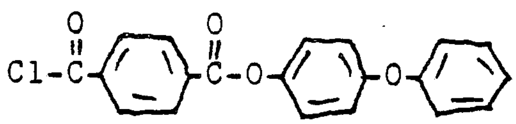

* * * * * *